# EUROPÄISCHE PATENTSCHRIFT

(11) **EP 2 456 747 B1**
(45) Veröffentlichungstag und Bekanntmachung des Hinweises auf die Patenterteilung: **25.06.2014**
(21) Anmeldenummer: 10732671.2
(22) Anmeldetag: 14.07.2010
(51) Int. Cl.: C07C 67/10, C07C 69/007

(54) **Verfahren zur Herstellung von Alkoxyenonen und Enaminoketonen**
Method for preparing alkoxyenones and enaminoketones
Procédé de fabrication des alkoxyénones et énaminocétones

(30) Priorität: 23.07.2009 EP 09166239
(43) Veröffentlichungstag der Anmeldung: 30.05.2012
(73) Patentinhaber: Bayer CropScience AG, 40789 Monheim (DE)
(72) Erfinder: PAZENOK, Sergii, 42699 Solingen (DE); LUI, Norbert, 51519 Odenthal (DE); NEEFF, Arnd, 51399 Burscheid (DE)
(74) Vertreter: BIP Patents
(86) Internationale Anmeldenummer: PCT/EP2010/004286
(87) Internationale Veröffentlichungsnummer: WO 2011/009552

(56) Entgegenhaltungen:
- DE-A1-102006 032 168
- K. V. TARASENKO ET AL: "Reactions of beta-aminovinyl bromodifluoromethyl ketones with alkyl phosphites: Perkow versus Arbuzov" COLLECT. CZECH. CHEM. COMMUN., Bd. 74, Nr. 2, 18. Februar 2009 (2009-02-18), Seiten 335-346, XP002555793 in der Anmeldung erwähnt
- M. A. P. MARTINS ET AL: "A convenient synthesis of 5-trichloromethyl-5-hydroxy-3-heteroalkyl- 4,5-dihydroisoxazoles" SYNTHESIS, Nr. 13, 2001, Seiten 1959-1964, XP002555794 in der Anmeldung erwähnt

## Beschreibung

Die vorliegende Erfindung betrifft und ein neues Verfahren zur Herstellung von Alkoxyenonen und Enaminoketonen Alkoxyenone und Enaminoketone sind wertvolle Zwischenprodukte zur Herstellung von Pyrazolen und Anthranilsäureamiden, die als Insektizide Verwendung finden können.

In der Literatur ist bekannt, dass 5-Bromo-1,1,1-trihalo-4-methoxypent-3-en-2-one drei Reaktionszentren (am Carbonylkohlenstoff, sowie am C4- und C5-Kohlenstoff) hat, welche je nach Nukleophileart und Bedingungen auf unterschiedliche Weise reagieren können. So beschreibt Gerus et al. (Coll.Czech Chem. Comm, 2009, v.74, N 2, p. 335-346), daß Alkoxyketone mit Triethylphosphiten unter der Bildung von "Arbuzov"-Produkten der Formel (IX) oder "Perkow" Produkten der Formel (X) reagieren (siehe nachfolgendes Schema (A)):

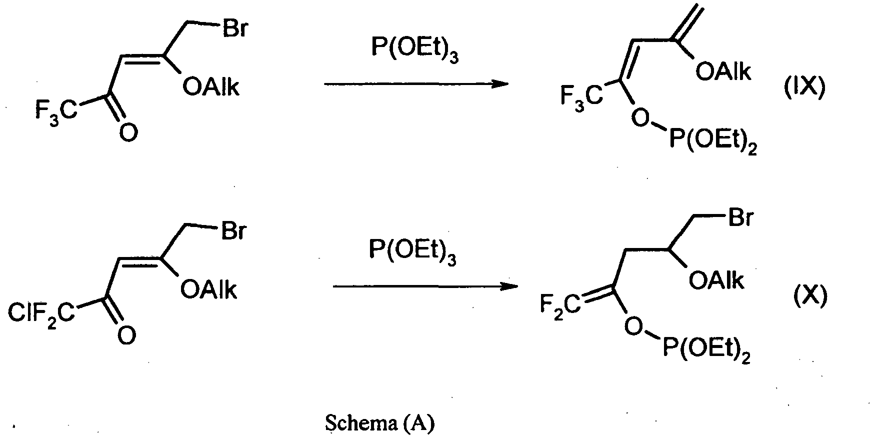

Die Aufgabe der Erfindung ist es, ein Verfahren zur Verfügung zu stellen, welches erlaubt, das Halogenatom in Position 5 beispielsweise der Verbindungen 5-Halo-1,1,1-trihalogen-4-(alkoxy)pent-3-en-2-one oder 5-Halo-1,1,1-trihalogen-4-(diakylamino)pent-3-en-2-one gegen O-Nukleophile selektiv auszutauschen, ohne die Trihalogenmethylgruppe am Carbonyl-Kohlenstoff anzugreifen. Das Verfahren soll für die technische Produktion des Produktes tauglich sein. Eine weitere Aufgabe der vorliegenden Erfindung ist es, ein Verfahren zu entwickeln, welches es erlaubt, Alkoxyenone und Enaminoketone in ausreichender Menge und Reinheit für die weitere Herstellung von Pyrazolen und Anthranilsäureamiden bereitzustellen.

Erfindungsgemäß gelöst wurde die Aufgabe durch ein Verfahren zur Herstellung der Alkoxyenone und Enaminoketone der Formel (I), 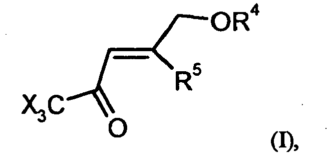 in welcher
- R⁴: -(C=O)Alkyl,
- X: für Halogen steht,
- R⁵: für Alkoxy, Dialkylamino, wobei die Alkylgruppen gegebenenfalls einen Ring bilden können, welcher gegebenenfalls 1-2 weitere Heteroatome aus der Reihe O,N,S enthalten kann, für Cycloalkylamino, Thioalkyl steht,
dadurch gekennzeichnet, dass die Verbindungen der allgemeinen Formel (III), 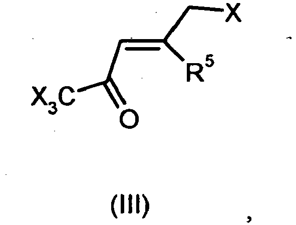 in welcher R⁵ die oben angegebenen Bedeutungen hat und X unabhängig voneinander für Fluor, Chlor, Brom oder Jod steht,
mit einer Verbindung der allgemeinen Formel (II)

KatOR⁴ (II),

in welcher

Kat für ein Kation steht, für Li, Na, K, Cs, (Alkyl)₄N, (Alkyl)₄P, (Aryl)₄P steht,
- R⁴: -(C=O)Alkyl
- R⁴: bevorzugt für -(C=O)(C₁-C₆)Alkyl, steht,
- R⁴: besonders bevorzugt für -(C=O)(C₁-C₆)Alkyl steht,
- R⁴: ganz besonders bevorzugt für -(C=O)CH₃ steht,
umgesetzt werden zu erfindungsgemäßen Alkoxyenonen und Enaminoketonen der Formel (I), welche zu Pyrazolen und Anthranilsäureamiden weiterverarbeitet werden können.

Das Verfahren liefert die Verbindungen der Formel (I) ohne Nebenreaktionen und weist somit die im Stand der Technik beschriebenen Nachteile nicht auf.

Insbesondere ist es als völlig überraschend anzusehen, dass 5-Halo-1,1,1-trihalogen-4-alkoxy- oder -(dialkylamino)pent-3-en-2-one mit den Verbindungen der Formel (II) regioselektiv reagiert und das Halogenatom in Position 5 gegen O-Nukleophile beispielsweise die OR⁴-Gruppe tauscht, ohne die Trihalogenmethyl-Gruppe X₃CO- anzugreifen.

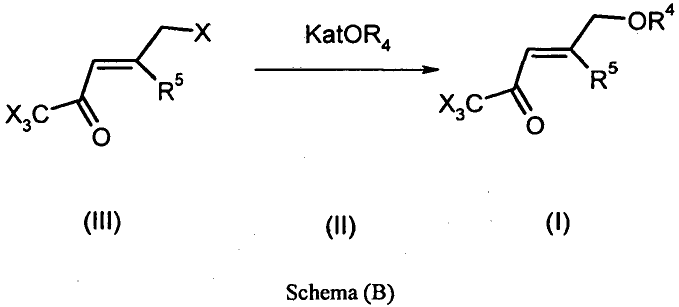

Wobei Kat, X, R⁴, R⁵ die oben angegebenen Bedeutungen haben.

### Allgemeine Definitionen

Im Zusammenhang mit der vorliegenden Erfindung, umfasst der Begriff Halogene (X), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden. Substituierte Gruppen können einfach oder mehrfach substituiert sein, wobei bei Mehrfachsubstitutionen die Substituenten gleich oder verschieden sein können.

Mit einem oder mehreren Halogenatomen (-X) substituierte Alkyl-Gruppen (= Halogenalkyl-Gruppen) sind beispielsweise ausgewählt aus Trifluormethyl (CF₃), Difluormethyl (CHF₂), CCl₃, CFCl₂, CF₃CH₂, ClCH₂, CF₃CCl₂.

Alkyl-Gruppen sind im Zusammenhang mit der vorliegenden Erfindung, soweit nicht abweichend definiert, lineare oder verzweigte Kohlenwasserstoff-Gruppen.

Die Definition Alkyl und C₁-C₁₂-Alkyl umfasst beispielsweise die Bedeutungen Methyl, Ethyl, n-, iso-Propyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Cycloalkyl-Gruppen sind im Zusammenhang mit der vorliegenden Erfindung, soweit nicht abweichend definiert, ringförmige gesättigte Kohlenwasserstoff-Gruppen, welche gegebenenfalls 1-3 Heteroatome aus der Reihe O,N,S enthalten können.

Aryl-Reste sind im Zusammenhang mit der vorliegenden Erfindung, soweit nicht abweichend definiert, C₆-C₁₀ aromatische Kohlenwasserstoff-Reste und aromatische Kohlenwasserstoff-Reste, die ein, zwei oder mehrere Heteroatome, die ausgewählt sind aus O, N, P und S aufweisen können und optional durch weitere Gruppen substituiert sein können.

Die erfindungsgemäßen Verbindungen können gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Es werden sowohl die E- als auch die Z-Isomere, wie auch die threo- und erythro-, und auch die optischen Isomere, beliebige Mischungen dieser Isomere, sowie die möglichen tautomeren Formen offenbart und beansprucht.

### Verbindungen der Formel (III)

Die bei der Durchführung des erfindungsgemäßen Verfahrens als Ausgangsstoffe verwendeten Verbindungen sind durch die Formel (III) allgemein definiert: 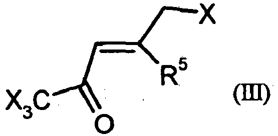 wobei
- X: unabhängig voneinander für Fluor, Chlor, Brom, Jod, bevorzugt für Fluor, Chlor oder Brom, besonders bevorzugt für Chlor oder Brom steht,
- R⁵: für Alkoxy, Dialkylamino, wobei die Alkylgruppen gegebenenfalls einen Ring bilden können, welcher gegebenenfalls 1-2 weitere Heteroatome aus der Reihe O,N,S enthalten kann, für Cycloalkylamino, Thioalkyl, steht, bevorzugt für Alkoxy, besonders bevorzugt für (C₁-C₆)Alkoxy steht.

5-Halo-1,1,1-trihalogen-4-methoxypent-3-en-2-one können nach der Methode von Gerus et al., Synthesis 2001, N. 3, 431-436, hergestellt werden.

Beispiele für erfindungsgemäß geeignete Verbindungen der Formel (III) sind z.B.:

5-Brom-1,1,1-trichloro-4-methoxypent-3-en-2-one, 5-chor-1,1,1-trichloro-4-methoxypent-3-en-2-one, 5-Bromo-1,1,1-trifluor-4-methoxypent-3-en-2-one, 5-Bromo-1,1,1-trichloro-4-ethoxypent-3-en-2-one, 5-Chlor-1,1,1-trifluor-4-methoxypent-3-en-2-one, 5-Chlor-1,1,1-trifluor-4-dimethylaminopent-3-en-2-one.

### Verbindungen der Formel (II)

Die bei der Durchführung des erfindungsgemäßen Verfahrens als Ausgangsstoffe verwendeten Verbindungen sind durch die Formel (II) allgemein defmiert

KatOR⁴ (II),

wobei Kat und R⁴ die oben angegebenen Bedeutungen haben.

Beispielhaft, aber nicht limitierend seien die folgenden Verbindungen der Formel (II) aufgeführt: NaOAc, KOAc, CsOAc.

Die Verbindungen der Formel (II) sind kommerziell erhältlich.

Die Durchführung des erfindungsgemäßen Verfahrensschritts erfolgt vorzugsweise innerhalb eines Temperaturbereichs von 10°C bis 100°C, besonders bevorzugt bei Temperaturen von 20°C bis 80 °C. Der erfindungsgemäße Verfahrensschritt wird im Allgemeinen unter Normaldruck durchgeführt.

Die Reaktionszeit ist nicht kritisch und kann, in Abhängigkeit von der Ansatzgröße und Temperatur, in einem Bereich zwischen 30 min. und mehreren Stunden gewählt werden.

Bei der Durchführung des erfmdungsgemäßen Verfahrensschritts setzt man 1 Mol des Haloenones der Formel (III) mit 0.8 Mol bis 1.5 Mol, vorzugsweise 0.9 Mol bis 1.2 Mol, besonders bevorzugt mit der äquimolaren Menge der Verbindung der Formel (II) um.

Geeignete Lösungsmittel sind beispielsweise aliphatische, alicyclische oder aromatische Kohlenwasserstoffe, wie z.B. Petrolether, n-Hexan, n-Heptan, Cyclohexan, Methylcyclohexan, Benzol, Toluol, Xylol oder Decalin, und halogenierte Kohlenwasserstoffe, wie z.B. Chlorbenzol, Dichlorbenzol, Dichlormethan, Chloroform, Tetrachlormethan, Dichlorethan oder Trichlorethan, Ether, wie Diethylether, Diisopropylether, Methyl-tert-butylether, Methyl-tert-amylether, Dioxan, Tetrahydrofuran, 1,2-Dimethoxyethan, 1,2-Diethoxyethan oder Anisol; Nitrile, wie Acetonitril, Propionitril, n- oder iso-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Sulfoxide, wie Dimethylsulfoxid oder Sulfone, wie Sulfolan, Alkohole wie Methanol, Ethanol, Isopropanol. Besonders bevorzugt verwendet man Acetonitril, Toluol, Ethanol, THF, Diglym, N-Methylpyrrolidon, 1,2-Diethoxyethan.

Die Umsetzung kann durch den Zusatz von Katalysatoren beschleunigt werden.

Als Katalysatoren können Substanzen eingesetzt werden, die allgemein Umsetzungen mit O-Nukleophilen beschleunigen. Beispielsweise können Alkalibromide oder Alkalijodide, wie zum Beispiel Natriumjodid, Kaliumjodid, Kaliumbromid, Ammoniumbromid oder Ammoniumjodid; Tetraalkylammoniumjodide, Tetraalkylammoniumbromide oder Tetraalkylammoniumsulphate, wie zum Beispiel Tetraethylammoniumchlorid, Tetraethylammoniumbromid, Tetrabutylammoniumchlorid oder Tetrabutylammoniumbromid; Tetraalkyl- oder Tetraarylphosphoniumchloride oder -bromide oder -jodide, wie zum Beispiel Bis(dimethylamino)[(1,3-dimethylimidazolidin-2-yliden)amino]methyliumbromid, Hexadecyl-tri-butyl-phosphoniumbromid, Stearyltributylphosphoniumbromid, Tetrabutylphosphoniumbromid, Tetra-butyl-phosphoniumbromid, Tetraoctylphosphoniumbromid, Tetraphenylphosphoniumchlorid, Tetraphenylphosphoniumbromid, Tetrakis(dimethylamino)phosphonium-bromid, Tetra-kis-(diethylamino)phosphoniumbromid, Tetrakis(dipropylamino)phosphoniumchlorid oder -bromid; Kronenether, beispielsweise 18-Krone-6 eingesetzt werden.

Die Menge des Katalysators kann von 0,1 bis 15 Gewichtsprozent, bezogen auf die eingesetzte Verbindung der Formel (II) variiert werden. Höhere Mengen an Katalysator sind möglich, aber nicht wirtschaftlich.

Martins et al. (Synthesis 2001, 13, 1959) beschreibt , dass 5-Bromo-1,1,1-trichlor-4-methoxypent-3en-2-one mit Phenol in Gegenwart von Pottasche bei 80°C in Acetonitril unter Br/OPh Austausch reagiert.

Im vorliegenden Fall ergab diese Umsetzung ein komplexes Gemisch von mehreren Produkten, wobei der Abbau der CCl₃-Gruppe dominierte.

Um den Haloformabbau (besonders im Fall von CCl₃-Enonen) zu vermeiden, wird zu dem Reaktionsgemisch die Säure zugesetzt und hierdurch die Basizität des Reaktionsgemisches abgesenkt. Dadurch wird die Bildung von Nebenprodukten durch Abbau der CCl₃-Gruppe signifikant zurückgedrängt. Die Menge der Säure kann von 0,05 bis I Äquivalent bezogen auf die eingesetzte Verbindung der Formel (III), variiert werden. Als Zusatz kommen HCl, H₂SO₄, CH₃COOH, CF₃COOH in Frage, wobei CH₃COOH, HCl und H₂SO₄ bevorzugt sind.

Die Aufarbeitung des Reaktionsgemisches erfolgt wasserfrei durch Befreiung des Gemisches von Salzen (Filtration) und Entfernung der Lösemittel im Vakuum. Wässrige Aufarbeitung ist auch möglich. Es ist auch möglich, das Gemisch ohne Zwischenisolierung weiter umzusetzen.

Die Reinheit der Verbindungen der Formel (I) ist sehr hoch und liegt in Bereich von 95-97 %, was es erlaubt, die Verbindungen ohne Reinigungsschritt weiter einzusetzen. Insbesondere zeichnet sich die erfindungsgemäße Umsetzung durch den Einsatz günstiger Rohstoffe, sowie durch eine auch im großtechnischen Maßstab besonders gut und einfach durchzuführende Prozeßführung aus.

Die Verbindungen der Formel (I) sind wichtige Zwischenprodukte für die Herstellung von Pyrazolen gemäß Schema (C), welche wiederum wertvolle Zwischenprodukte sind in der Synthese von Anthranilsäureamiden (WO2007/112893 WO2007/144100). 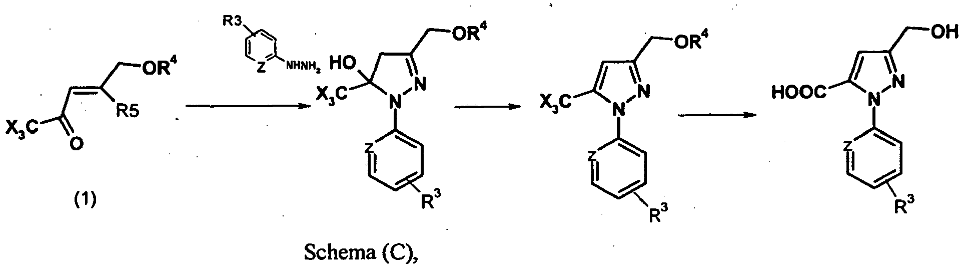 wobei
- Z: für CH, N steht,
- R³: für H, Halogen, CN, NO₂, Alkyl, Cycloalkyl, Halogenalkyl, Halogencycloalkyl, Alkoxy, Halogenalkoxy, Alkylamino, Dialkylamino, Cycloalkylamino steht und

R⁴, R⁵ und X die oben angegebenen Bedeutungen haben.

### Herstellbeispiele

### Beispiel 1

*5-Bromo-1,1,1-trichloro-4-methoxypent-3-en-2-one* wurde hergestellt nach der Methode von Gerus et al., Synthesis 2001, N. 3, 431-436. Ausbeute 90 %.

### Beispiel 2

### 5,5,5-Trichloro-2-methoxy-4-oxopent-2-en-1-yl acetate

29.6 g (0.1 mol) 5-Bromo-1,1,1-trichloro-4-methoxypent-3-en-2-one, 17 g Kaliumacetate, 5 g Tetrabutylammoniumbromid und 8 g Essigsäure wurden in 300 ml Acetonitril 16 Std. bei 40°C gerührt. Das Gemisch wurde im Vakuum aufkonzentriert und zu dem Rückstand wurde Wasser zugegeben. Das Produkt wurde mit Ethylacetat extrahiert, die organische Phase mit Wasser gewaschen und das Lösemittel im Vakuum vollständig entfernt.

Man erhielt 25.4 g (85%) des Produktes als hell-braunen Feststoff mit der LC Reinheit von 97 %, Schmp. 53-55°C.

¹H NMR (DMSO d₆) δ: 2.05 (s, 3H), 3.85 (s, 3H), 5.2 (s, 2H), 6.1 (s, 1H) ppm.

GC/MS: m/z 274.

### Beispiel 3

### 5,5,5-Trichloro-2-methoxy-4-oxopent-2-en-1-yl acetate

29.6 g (0.1 mol) 5-Bromo-1,1,1-trichloro-4-methoxypent-3-en-2-one, 19.1 g Cesiumacetate, 5 g Tetrabutylammoniumbromid und 8 g Essigsäure wurden in 300 ml Acetonitril 8 Std. bei 25°C gerührt. Der Niederschlag wurde abfiltriert und die organische Phase im Vakuum eingeengt.

Man erhielt 26.8 g (90 %) des Produktes als hell-braunen Feststoff mit der LC Reinheit von 98 %. Schmp. 54-55°C.

### Beispiel 4

### 5,5,5-Trifuor-2-methoxy-4-oxopent-2-en-1-yl acetate

24.8 g (0.1 mol) 5-Bromo-1,1,1-trifluor-4-methoxypent-3-en-2-one, 20 g Kaliumacetate, 5 g Tetrabutylammoniumbromid und 8 g Essigsäure wurden in 300 ml Acetonitril 20 Std. bei 40°C gerührt. Der Niederschlag wurde abfiltriert und das Lösemittel im Vakuum vollständig entfernt.

Man erhielt 15.8. g (70%) als hell-braunes Öl mit der Reinheit von 85 %.

¹H NMR (DMSO d₆) δ: 2.17 (s, 3H), 3.80 (s, 3H), 5.28 (s, 2H), 6.15 (s, 1H) ppm. GC/MS: m/z 226.

### Beispiel 4 b

### [1-(3-Chloropyridin-2-yl)-5-(trichloromethyl)-1H-pyrazol-3-yl]methyl acetate.

38.7 g [1-(3-Chloropyridin-2-yl)-5-hydroxy-5-(trichloromethyl)-4,5-dihydro-1H-pyrazol-3-yl]methyl acetate wurden in 200 ml Methyltert-butylether gelöst und 12.6 g Oxalylchlorid wurden binnen 2 Std. zugetropft (starke Gasentwicklung). Das Gemisch wurde 5 Std. bei 25°C nachgerührt und im Vakuum komplett eingeengt.

Man erhielt 36 g des Produktes als zähes Öl, welches nach ca. 10 Std. bei Raumtemperatur durchkristallisiert. Schmp. 40-42°C.

¹H NMR (DMSO d₆) δ: 2.0 (s, 3H), 5.1 (dd, 2H), 7.0 (s, 1H), 7.6 (dd,1H), 8.1 (dd, 1H), 8.5 (dd, 1H) ppm.

### Beispiel 5

### [1-(3-Chloropyridin-2-yl)-5-hydroxy-5-(trichloromethyl)-4,5-dihydro-1H-pyrazol-3-yl]methyl acetate

27.5 g (0.1 mol) 5,5,5-Trichloro-2-methoxy-4-oxopent-2-en-1-yl acetate und 14.4 g (0.1 mol) 3-Chlor-2-hydrazinopyridine wurden in 200 ml Ethanol vorgelegt und das Gemisch 3 Std. bei 25°C gerührt. Der Niederschlag wurde abfiltriert und mit Cyclohexan gewaschen.

Man erhielt 34 g des Produktes (90 % Ausbeute) als weißen Feststoff mit einem Schmelzpunkt von 105-106°C.

¹H NMR (DMSO d₆) δ: 2.07 (s, 3H), 3.30 (dt, 1H), 3.78 (dt, 1H), 4.79 (dt, 1H), 4.84 (dt, 1H), 7.23 (dd, 1H), 7.95 (dd, 1H), 8.22 (dd, 1H), 9.46 (br.s, 1H) ppm.

### Beispiel 5 b

### [1-(3-Chloropyridin-2-yl)-5-(trichloromethyl)-1H-pyrazol-3-yl]methanol

36.9 g [1-(3-Chloropyridin-2-yl)-5-(trichloromethyl)-1H-pyrazol-3-yl]methyl acetate wurden in 100 ml Ethanol gelöst und 20 g NaOH (als 40 % Lösung in Wasser) zugegeben. Nach 1 Std. wurde das Gemisch mit 300 ml Wasser verdünnt, das Produkt wurde abfiltriert, mit Wasser gewaschen und getrocknet. Man erhielt 30 g (95 %) des Produktes als weißen Feststoff.

Schmp. 109-111°C.

¹H NMR (DMSO d₆) δ: :4.55 (2H); 6.95 (1H); 7.55 (dd, 1H); 8.05 (dd, 1H); 8.5 (dd, 1H) ppm.

### Beispiel 6

### Hydrochlorid von 1-(3-chloropyridin-2-yl)-3-(hydroxymethyl)-1H-pyrazole-5-carboxylic acid

38.7 g (0.1 mol) [1-(3-Chloropyridin-2-yl)-5-(trichloromethyl)-1H-pyrazol-3-yl]methanol und 10 g H₂SO₄ (als 10 % Lösung in Wasser) wurden 3 Std. bei 80°C gerührt.

Das Gemisch wurde auf 0°C abgekühlt; der Niederschlag wurde abfiltriert und mit Acetonitril gewaschen und getrocknet.

Ausbeute 90 %. Schmp. 173-175°C.

¹H NMR (DMSO d₆) δ: 3,5 (b.s, 1H) 4.50, (2H); 5.2 (b. s), 6.95 (1H); 7.55 (dd, 1H); 8.05 (dd, 1H); 8.5 (dd, 1H), 13 (b.s) ppm.

### Beispiel 7

### 2-[5-carboxy-3-(hydroxymethyl)-1H-pyrazol-1-yl]-3-chloropyridinium hydrochloride

Man arbeitet wie in Beispiel 6 beschrieben, nimmt jedoch

### [1-(3-Chloropyridin-2-yl)-5-(trichlormethyl)-1H-pyrazol-3-yl]methyl acetate

Ausbeute 95 %. Schmp. 173-175°C.

### Beispiel 8

### 3-[(Benzyloxy)methyl]-1-(3-chloropyridin-2-yl)-1H-pyrazole-5-carboxylic acid

4.4 g 2-{3-[(Benzyloxy)methyl]-5-(trichloromethyl)-1H-pyrazol-1-yl}-3-chloropyridine und 30 ml 20 % H₂SO₄ wurden 24 Std. bei 100°C erhitzt.

Der Niederschlag wurde abfiltriert und mit Wasser gewaschen. Die Ausbeute betrug 92 %.

### Charakterisierung:

¹H NMR (CDCl3) δ: 4.61 (2H, s); 4.63 (m, 2H), 6.97 (1H, s); 7.2-7.4 (5H, m); 7.42 (1H,m); 7.96 (1H,d 2 Hz.); 8.5 (1H, d, 2 Hz) ppm.

### Beispiel 9

### 1-(3-chloropyridin-2-yl)-3-(hydroxymethyl)-1H-pyrazole-5-carboxylic acid hydrochlorid

3.43 g 3-[(Benzyloxy)methyl]-1-(3-chloropyridin-2-yl)-1H-pyrazole-5-carboxylic acid und 20 ml HCl (37,5 %) wurden 2 Std. bei 100°C erhitzt und dann das Reaktionsgemisch im Vakuum bei 10 mbar komplett eingeengt. Man erhielt 1-(3-chloropyridin-2-yl)-3-(hydroxymethyl)-1H-pyrazole-5-carboxylic acid als Hydrochlorid. Durch Neutralisation mit NaHCO₃ erhielt man die freie Säure als weißen Feststoff.. Die Ausbeute betrug 94 %.

### Beispiel 10

### Methyl 3-(chlormethyl)-1-(3-chloropyridin-2-yl)-1H-pyrazole-5-carboxylate

1-(3-Chloropyridin-2-yl)-3-(hydroxymethyl)-1H-pyrazole-5-carboxylic acid hydrochlorid (0.1 mol) wurde in 100 ml Toluol vorgelegt. 24 g von SOCl₂ wurde portionsweise bei 60°C zugegeben. Man erhitzte das Gemisch 3 Std. bei 70°C, wobei der Niederschlag komplett in die Lösung ging. Methanol (30 ml) wurde langsam zu dem Gemisch zugetropft und die Lösung 1 Std. bei 30°C nachgerührt. Anschließend wurde die Lösung im Vakuum eingeengt. Man erhielt 95% des Produktes mit einer Reinheit von 96 % .

### Charakterisierung

¹H NMR (CDCl₃) δ: 3.7 (3H,s); 4.7 (2H, s); 7.1 (1H, s); 7.5 (1H, m); 8.05 (1H, m); 8.5 (1H, m) ppm.

### Beispiel 11

### Methyl 1-(3-chloropyridin-2-yl)-3-(hydroxymethyl)-1H-pyrazole-5-carboxylate

32.6 g [1-(3-chloropyridin-2-yl)-5-(trichloromethyl)-1H-pyrazol-3-yl]methanol und 300 ml Methanol wurden 3 Std. bei 90°C im Autoklaven erhitzt. Methanol wurde im Vakuum entfernt und der Niederschlag mit Wasser gewaschen. Ausbeute 25 g 94%. Schmp. 104°C.

### Beispiel 12

### Methyl 3-(chloromethyl)-1-(3-chloropyridin-2-yl)-1H-pyrazole-5-carboxylate

Methyl 1-(3-chloropyridin-2-yl)-3-(hydroxymethyl)-1H-pyrazole-5-carboxylate (26.7 g, 0.1 mol) wurde in 150 ml CH₂Cl₂ gelöst und die Lösung auf 5°C gekühlt. SOCl₂ (0.12 mol) in 30 ml CH₂Cl₂ wurde langsam bei dieser Temperatur zugetropft. Das Gemisch wurde 4 Std. bei 40°C nachgerührt und im Vakuum eingeengt. Das Produkt kann ohne Reinigung weiter eingesetzt werden.

### Analytische Charakterisierung

¹H NMR (CD₃CN) δ: 8,52 (1H,d); 8,06 (1H,d), 7,55 (1H, dd); 7,10 (1H, s); 4,75 (2H,s); 3,75 (3H,s) ppm.

## Patentansprüche

1. Verfahren zur Herstellung von Verbindungen der allgemeinen Formel (I), 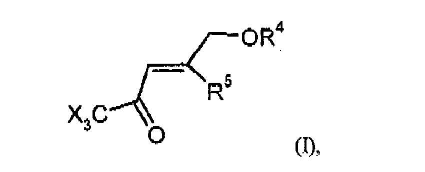 in welcher
R⁴ für-(C=O)Alkyl steht,
X für Halogen steht,
R⁵ für Alkoxy, Dialkylamino, wobei die Alkylgruppen gegebenenfalls einen Ring bilden können, welcher gegebenenfalls 1-2 weitere Heteroatome aus der Reihe O,N,S enthalten kann, für Cycloalkylamino, Thioalkyl steht,
**dadurch gekennzeichnet, dass** die Verbindungen der allgemeinen Formel (III), 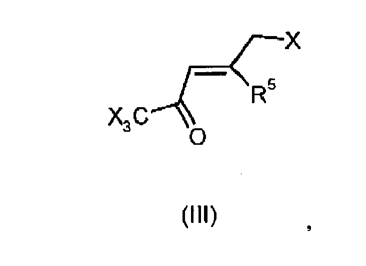 in welcher R⁵ die oben angegebenen Bedeutungen hat und X unabhängig voneinander für Fluor, Chlor, Brom oder Jod steht,
mit einer Verbindung der allgemeinen Formel (II)
KatOR⁴ (II),
in welcher
Kat für ein Kation steht, für Li, Na, K, Cs, (Alkyl)₄N, (Alkyl)₄P, (Aryl)₄P steht,
R⁴ für -(C=O)Alkyl steht,
umgesetzt werden zu Verbindungen der Formel (I).

2. Verfahren gemäß Anspruch 1, **dadurch gekennzeichnet, dass**
R⁴ für -(C=O)(C₁-C₆)Alkyl steht,
X für Chlor oder Brom steht,
R⁵ für (C₁-C₆)Alkoxy steht

3. Verfahren gemäß einem der Ansprüche 1 oder 2, **dadurch gekennzeichnet, dass** zusätzlich eine Säure zugesetzt wird.

4. Verfahren gemäß einem der Ansprüche 1, 2 oder 3, **dadurch gekennzeichnet, dass** die Säure ausgewählt ist aus HCl, H₂SO₄, CH₃COOH oder CF₃COOH.

5. Verfahren gemäß einem der Ansprüche 1 bis 4, **dadurch gekennzeichnet, dass** ein Katalysator eingesetzt wird.

6. Verfahren gemäß einem der Ansprüche 1 bis 5, dadurch gekennzeichet, dass als Säure CH₃COOH und als Katalysator Tetrabutylammoniumbromid eingesetzt wird.

## Claims

1. Process for preparing compounds of the general
formula (I), 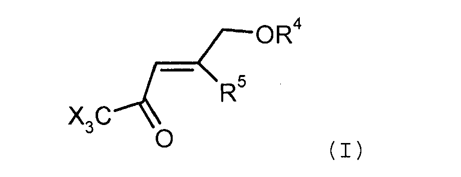 in which
R⁴ is -(C=O)alkyl,
X is halogen,
R⁵ is alkoxy, dialkylamino where the alkyl groups may optionally form a ring which may optionally contain 1-2 further heteroatoms from the group of O, N, S, or is cycloalkylamino, thioalkyl,
**characterized in that** the compounds of the general formula (III) 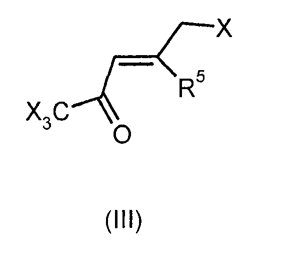 in which R⁵ is as defined above and X is independently fluorine, chlorine, bromine or iodine,
are reacted with a compound of the general formula (II)
KatOR⁴ (II)
in which
Kat is a cation, and is Li, Na , K, Cs, (alkyl)₄N, (alkyl)₄P, (aryl)₄P,
R⁴ is -(C=O) alkyl,
to give compounds of the formula (I).

2. Process according to Claim 1, **characterized in**
**that**
R⁴ is -(C=O)(C₁-C₆)alkyl,
X is chlorine or bromine,
R⁵ is (C₁-C₆)alkoxy.

3. Process according to either of Claims 1 and 2, **characterized in that** an acid is additionally added.

4. Process according to any of Claims 1, 2 or 3, **characterized in that** the acid is selected from HCl, H₂SO₄, CH₃COOH and CF₃COOH.

5. Process according to any of Claims 1 to 4, **characterized in that** a catalyst is used.

6. Process according to any of Claims 1 to 5, **characterized in that** the acid used is CH₃COOH and the catalyst tetrabutylammonium bromide.

## Revendications

1. Procédé pour la préparation de composés de formule générale (I), 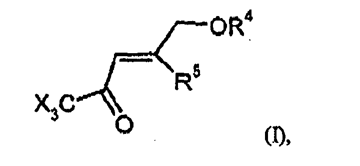 dans laquelle
R⁴ représente -(C=O)alkyle,
X représente halogène,
R⁵ représente alcoxy ; dialkylamino, les groupes alkyle pouvant le cas échéant former un cycle, qui peut le cas échéant contenir 1-2 autres hétéroatomes de la série O, N, S ; cycloalkylamino ; thioalkyle ;
**caractérisé en ce qu'**on transforme des composés de formule générale (III) 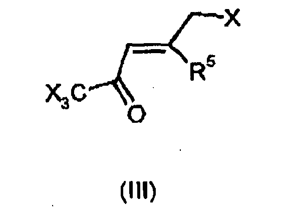 dans laquelle R⁵ présente les significations indiquées ci-dessus et X représente, indépendamment l'un de l'autre fluor, chlore, brome ou iode, avec un composé de formule générale (II)
KatOR⁴ (II),
dans laquelle
Kat représente un cation, représente Li, Na, K, Cs, (alkyl)₄N, (alkyl)₄P, (aryl)₄P,
R⁴ représente -(C=O)alkyle,
en composés de formule générale (I).

2. Procédé selon la revendication 1, **caractérisé en ce que**
R⁴ représente - (C=O) (C₁-C₆)alkyle,
X représente chlore ou brome,
R⁵ représente (C₁-C₆)alcoxy.

3. Procédé selon l'une quelconque des revendications 1 ou 2, **caractérisé en ce qu'**on ajoute en plus un acide.

4. Procédé selon l'une quelconque des revendications 1, 2 ou 3, **caractérisé en ce que** l'acide est choisi parmi HCl, H₂SO₄, CH₃COOH ou CF₃COOH.

5. Procédé selon l'une quelconque des revendications 1 à 4, **caractérisé en ce qu'**on utilise un catalyseur.

6. Procédé selon l'une quelconque des revendications 1 à 5, **caractérisé en ce qu'**on utilise, comme acide, du CH₃COOH et comme catalyseur, du bromure de tétrabutylammonium.
